# FASCICULE DE BREVET EUROPEEN

(11) **EP 3 573 513 B1**
(45) Date de publication et mention de la délivrance du brevet: **24.03.2021**
(21) Numéro de dépôt: 17829225.6
(22) Date de dépôt: 27.12.2017
(51) Int. Cl.: B60K 28/06, B60W 50/14, B60W 40/08, A61B 5/18, A61B 5/00

(54) **SÉCURITÉ CONCERNANT UN ENGIN ET UNE PERSONNE ÉQUIPÉE D'UN DISPOSITIF MÉDICAL**
SICHERHEIT FÜR EINE MASCHINE UND EINE PERSON MIT EINEM MEDIZINPRODUKT
SAFETY RELATING TO A MACHINE AND TO A PERSON FITTED WITH A MEDICAL DEVICE

(30) Priorité: 29.12.2016 FR 1670800
(43) Date de publication de la demande: 04.12.2019
(73) Titulaire: Chaumeil, Arnaud, 31300 Toulouse (FR)
(72) Inventeur: Chaumeil, Arnaud, 31300 Toulouse (FR)
(74) Mandataire: Warusfel, Bertrand
(86) Numéro de dépôt international: PCT/FR2017/000260
(87) Numéro de publication internationale: WO 2018/122474

(56) Documents cités:
- WO-A1-2016/070981
- DE-A1-102014 206 960
- GB-A- 2 534 471

## Description

L'invention concerne la sécurité concernant un engin et une personne équipée d'un dispositif médical.

L'invention a plus spécialement pour objet, en premier lieu, un procédé de sécurité concernant un engin comportant des moyens d'entraînement et au moins un poste pour une personne, dont au moins un poste de service pour une personne servant l'engin - notamment le commandant -, et au moins une personne équipée d'au moins un dispositif médical, l'engin étant conçu pour qu'au moins une personne équipée lui soit associé en se trouvant à un poste de l'engin - notamment au poste de service en vue de servir l'engin -. L'invention a pour objet, en deuxième lieu, un ensemble de sécurité spécialement destiné à la mise en œuvre du procédé, comprenant un dispositif médical équipant une personne et un module de sécurité. L'invention a pour objet, en troisième lieu, un tel dispositif médical et un tel module de sécurité spécialement destinés, l'un comme l'autre, à la mise en oeuvre du procédé et, ce faisant, à faire partie de l'ensemble de sécurité.

L'invention s'applique au cas d'un engin tel qu'un véhicule terrestre - comme une automobile, un train, un tracteur, un engin de manutention -, marin - comme un bateau -, aérien - comme un aéronef -, ou spatial. L'engin peut aussi être par exemple une grue.

Un tel engin comporte de façon connue en soi des moyens d'entraînement comme un groupe de motorisation ou de propulsion auquel sont associés les moyens usuels correspondant au type d'engin concerné, comme notamment des roues et des moyens de transmission pour un véhicule terrestre. Il comporte également un ou plusieurs postes pour une ou plusieurs personnes, dont un poste de service (ou éventuellement plusieurs postes de service) pour une personne (ou éventuellement plusieurs personnes) servant l'engin. Par « poste », il faut comprendre par exemple un siège et les moyens usuels associés. Ainsi, le poste de service comprend des organes de pilotage comme une télécommande, un volant, des manettes, des boutions, des pédales, etc. Par « servant » l'engin, il faut comprendre le commander, comme le piloter. Par convention, la personne se trouvant au poste de service est dénommée « pilote ». L'engin peut comporter également un ou plusieurs éventuels autres postes autres que de service, pour une ou plusieurs personnes que l'on qualifie ici, par convention, de « passagers ». Une personne se trouvant à un poste de l'engin est qualifiée d'«associée » à l'engin.

Par exemple, dans le cas d'un véhicule terrestre comme une automobile, pour une période de déplacement, le pilote, ici le conducteur de l'automobile, s'installe puis reste assis sur le siège du conducteur de sorte à manoeuvrer les organes de pilotages. Ceci est vrai même dans le cas d'une automobile ayant un certain degré d'autonomie. Entre les périodes de déplacement, lorsque l'automobile est à l'arrêt et n'est pas utilisée, le conducteur quitte l'automobile pour d'autres occupations. Il cesse alors d'être associé à l'automobile, conformément au sens donné en l'espèce au terme « associé », tout en restant éventuellement associable à elle plus tard. Dans certains cas, un conducteur donné peut utiliser des automobiles différentes, pour des périodes plus ou moins espacées, ou bien le conducteur n'a pas d'automobile attitrée, comme dans le cas de la location de véhicule. Ce qui vient d'être exposé pour une automobile peut être transposé et adapté à d'autres types de véhicules ou d'engins.

Dans le contexte de l'invention, une personne associable ou associée à l'engin (pour se trouver à un poste de celui-ci) est équipée d'un dispositif médical. Par convention, une telle personne est qualifiée de « personne équipée ».
Le cas échéant, il est prévu plusieurs personnes associables ou associées à l'engin équipées de dispositifs médicaux.

Il se peut également qu'une personne associable ou associée soit équipée de plusieurs dispositifs médicaux. En particulier, la personne équipée est celle qui est associable ou associée au poste de service pour servir l'engin, c'est-à-dire le pilote.

Dans le contexte de l'invention, un tel dispositif médical est conçu pour fonctionner en réponse à des données physiologiques de la personne équipée et pallier en tout ou partie un état médical problématique de la personne équipée. Selon les cas, un tel dispositif médical est implanté sur la personne équipée ou situé en externe. Et ce dispositif médical peut être visée thérapeutique et/ou diagnostique. On peut citer comme dispositif médical entrant dans le cadre de l'invention un stimulateur cardiaque, une prothèse, une pompe, un cœur artificiel, un exosquelette, cette liste n'étant pas limitative.

Dans le contexte de l'invention, il faut comprendre par « sécurité », une situation aussi objective que possible, reposant sur des conditions d'ordre médical, qui entraîne l'absence ou la minimisation de dangers ou de menaces.

Dans le contexte de l'invention, de tels dangers ou menaces concernent tout à la fois la personne équipée du dispositif médical, le dispositif médical en soi, l'engin ou encore des personnes ou des entités matérielles pouvant être impactées par l'engin.

Les dangers ou menaces concernant la personne équipée du dispositif médical sont que son état médical soit plus problématique que celui espéré dans des conditions prévisibles maîtrisées dans lesquelles le dispositif médical remplit sa fonction comme il est prévu. Un tel état plus problématique peut en effet être un danger ou une menace pour la vie même de la personne équipée ou bien au regard d'une dégradation grave ou irrémédiable de son état médical. Dans le cas où la personne équipée est le pilote, les dangers ou menaces sont que le pilote cesse d'être en situation de piloter convenablement l'engin, avec toutes les conséquences potentielles inhérentes.

Les dangers ou menaces concernant le dispositif médical sont que ses performances ou pire son fonctionnement même soient affectés, par exemple du fait d'un organe défectueux structurellement ou fonctionnellement. Un exemple typique est un état de charge déficient d"une batterie d'alimentation ou une déficience de montage.

Concernant l'engin les dangers ou menaces s'entendent de ceux concernant l'engin en tant que ses moyens d'entraînement fonctionnent. En effet, si l'engin n'est plus piloté convenablement l'engin, il existe un sérieux risque d'accident et de détérioration de l'engin. Et en cas d'accident, il existe un sérieux risque que l'engin impacte des personnes ou des entités matérielles se trouvant sur la trajectoire de l'engin.

Dans le contexte de l'invention, il faut comprendre par « alerte », un signal représentatif d'un danger ou d'une menace et pouvant être perçu comme tel par une - ou des - personnes et/ou une - ou des - entités matérielles.

Dans le contexte de l'invention, il peut être envisagé non pas une alerte unique, mais une pluralité d'alertes correspondant à des dangers ou menaces plus ou moins élevés. Dans ce cas, à une telle alerte est associé un indice du niveau d'alerte.

Un dysfonctionnement ou un problème source de danger ou de menace peut être avéré et effectif. Il peut n'être que seulement probable et à venir, étant alors le plus souvent précédé de signes annonciateurs qui doivent également être considérés comme cause d'alerte.

Si la question de la sécurité se pose avec l'acuité la plus grande pour la pilote de l'engin, elle se pose également pour un passager. En effet, l'état médical problématique d'un passager peut, à l'instar de celui du pilote, être un danger ou une menace pour sa vie même ou bien au regard d'une dégradation grave ou irrémédiable de son état médical. De plus, il ne fait pas de doute que ce qui affecte un passager est de nature à affecter le pilote. Aussi, la question de la sécurité se pose in fine pour toutes les personnes associées à l'engin.

De toutes les raisons qui viennent d'être exposées, il ressort que la sécurité concernant un engin et une personne équipée d'un dispositif médical est un problème crucial.

Ce problème est d'autant plus crucial que le nombre de personnes équipées d'un dispositif médical ira croissant, mais également parce qu'il est souhaitable que les interdictions ou limitations d'usage d'un engin compte tenu qu'une personne associable à lui soit équipée d'un dispositif médical soient fixées à bon escient. Des interdictions ou limitations d'usage trop strictes et fréquentes seraient excessives dès lors que la survenance d'un danger ou d'une menace serait a priori exceptionnelle et que les dispositifs médicaux sont de plus en plus performants et adaptés à chaque personne équipée. Inversement, des interdictions ou limitations d'usage pas assez strictes seraient inacceptables dès lors que la sécurité préventive est considérée comme un besoin essentiel et normal.

Le document US8423205 décrit un système de sécurité comprenant un implant, un appareil de commande et un véhicule. Le véhicule présente une unité de commande qui est une partie de son électronique de commande. Cette unité de commande est conçue pour permettre le fonctionnement d'un véhicule uniquement en cas de réception d'une identification unique et pour régler un mode de fonctionnement du véhicule en fonction d'un indice d'aptitude reçu, qui décrit l'aptitude d'un porteur d'implant à se servir d'un véhicule. L'implant est conçu pour saisir des données physiologiques d'un porteur d'implant. L'appareil de commande comprend une première interface pour une communication sans fil avec l'implant et une seconde interface pour une communication sans fil avec l'unité de commande du véhicule. L'appareil de commande comprend aussi une mémoire pour une identification unique. Dans une première réalisation, l'implant est conçu pour générer un indice d'aptitude à partir des données physiologiques saisies et pour transmettre l'indice d'aptitude à l'appareil de commande, tandis que la première interface est conçue pour recevoir l'indice d'aptitude et la seconde interface est conçue pour transmettre à l'unité de commande l'indice d'aptitude avec l'identification unique. Dans une seconde réalisation, l'implant est conçu pour transmettre les données physiologiques et l'appareil de commande comporte une unité de classification destinée à classer les données physiologiques dans une ou plusieurs classes d'aptitude.

Le système de sécurité décrit dans le document US8423205 présente nombre d'inconvénients et de limites.

Tout d'abord, ce système a pour seul but d'adapter la commande du véhicule à l'état du pilote. Par suite, ce système s'intéresse essentiellement au véhicule et ensuite aux personnes ou aux entités matérielles se trouvant sur la trajectoire du véhicule. En revanche, ce système ne s'intéresse pas directement au pilote équipé du dispositif médical. D'autre part, il ne s'intéresse pas aux passagers du véhicule. Enfin, il ne s'intéresse pas non plus au dysfonctionnement du dispositif médical en soi.

Ensuite, ce système est fondé sur l'existence d'une identification unique, laquelle soit être enregistrée dans le véhicule auquel le système est totalement intégré. Par suite, le véhicule est forcément dédié à une seule personne, pour autant qu'elle ait chargé le système associé au véhicule avec l'identification unique. Ce faisant, ce système n'est pas adapté aux cas ou le conducteur a vocation à utiliser des automobiles différentes, pour des périodes plus ou moins espacées, ou bien ou le conducteur n'a pas d'automobile attitrée, comme dans le cas de la location de véhicule. De plus, une telle identification unique peut être piratée avec toutes les conséquences qui en résultent.

Ensuite, si, dans une première réalisation de ce système, il est prévu que l'implant génère un indice d'aptitude à partir des données physiologiques saisies et transmette cet indice d'aptitude à l'appareil de commande, dans une seconde réalisation, il est prévu que l'implant transmette en clair les données physiologiques alors que l'appareil de commande comporte une unité de classification destinée à classer les données physiologiques dans une ou plusieurs classes d'aptitude, ce qui est très problématique. En effet, ces données physiologiques peuvent être piratées et leur confidentialité n'est pas assurée.

Les documents US 9 144 389 et US 9 332 910 décrivent des systèmes de sécurité comprenant un implant, un appareil de commande et un véhicule, dans lequel on transmet au véhicule des données physiologiques sur le conducteur.

Les documents EP 0 560 351, WO 2015/160272 décrivent des systèmes de sécurité fondés sur la détection de la présence ou de la fatigue du conducteur.

Le document WO 2015/138416 décrit un système de sécurité fondé sur une détection intégrée au siège du conducteur. Et les documents US 2010/0222687 et US 2005/0148894 sur une détection intégrée à la ceinture de sécurité du conducteur.

L'état de la technique comporte également les documents US 7 894 476 et US 6 480 744.

Aucun des systèmes précédemment mentionné pallie les inconvénients et limites du système de sécurité décrit dans le document US8423205, bien au contraire, et satisfait les exigences que l'on peut souhaiter pour une sécurité efficace et adaptée à l'époque concernant un engin et une personne équipée d'un dispositif médical.

Le document WO2016/070981 décrit un système et un procédé de surveillance de l'état de santé d'un occupant de véhicule. Le système comprend une unité de contrôle qui comprend: un récepteur pour la réception sans fil de paramètres physiologiques d'au moins une unité portée sur le corps et comprenant un ou plusieurs capteurs pour déterminer un ou plusieurs paramètres physiologiques de l'occupant du véhicule, et un module de diagnostic qui est conçu pour dériver des informations concernant l'état de santé, l'état de bien-être ou les incidents de maladie au moins partiellement mais obligatoirement sur la base des paramètres physiologiques reçus. L'unité de commande est également conçue pour fournir à l'occupant du véhicule, au moyen d'au moins une unité de sortie du véhicule, des informations concernant l'état de santé, et initier au moins l'une des étapes suivantes : adapter les fonctions du véhicule à l'état, ou, au moyen d'au moins une unité de sortie du véhicule, proposer ou effectuer de manière interactive des mesures qui améliorent l'état. A l'instar de ce qui a été précédemment exposé, ce système et ce procédé ne s'intéressent pas au dysfonctionnement de l'unité de contrôle en soi, laquelle unité de contrôle est un dispositif médical. Et ce système et de procédé visent à ce que le module de diagnostic reçoive et traite les données physiologiques de la personne concernée, ce qui est très problématique. Non seulement le recueil de toutes ces données n'est pas nécessaire aux fins de sécurité, mais elles peuvent être piratées et leur confidentialité n'est pas assurée.

Le document GB 2534471 décrit un procédé comprend l'étape consistant à fournir un protocole de communication pour une communication sans fil entre une télécommande mobile et un système de commande d'un véhicule. Un tel procédé ne permet pas de répondre au problème crucial de la sécurité concernant un engin et une personne équipée d'un dispositif médical qui s'y trouve. Ce procédé vise à prendre le contrôle à distance du véhicule, ce qui n'est pas le but de l'invention. En outre, ce procédé n'est pas polyvalent car il est associé à un véhicule donné. L'analyse d'un possible dysfonctionnement de la télécommande décrit dans le document ne peut se faire que parce que la télécommande et le véhicule sont indissociables.

Le document DE102014206960 décrit en relation avec un procédé permettant d'éviter les accidents ou la réduction de l'épisode d'accident, que des signes vitaux du conducteur sont déterminées dans un appareil médical mobile porté par le conducteur qui communique directement ou indirectement avec un système de sécurité dans le véhicule. Dans le cas où les signes vitaux sont en dehors d'une plage normale, une procédure automatique est exécutée dans le système de sécurité. Comme précédemment, ce procédé transmet obligatoirement et analyse les données physiologiques du conducteur, ce qui est très problématique. Et il ne s'intéresse pas au dysfonctionnement de l'appareil médical lui-même.

Par suite, il existe le besoin, d'une part, d'un procédé de sécurité concernant un engin comportant des moyens d'entraînement et au moins un poste pour une personne, dont au moins un poste de service pour une personne servant l'engin - notamment le commandant -, et au moins une personne équipée d'au moins un dispositif médical, l'engin étant conçu pour qu'au moins une personne équipée lui soit associé en se trouvant à un poste de l'engin - notamment au poste de service en vue de servir l'engin -, d'autre part, d'un ensemble de sécurité spécialement destiné à la mise en oeuvre d'un tel procédé, qui satisfont les exigences que l'on peut souhaiter pour une sécurité efficace et adaptée à l'époque.

Ces exigences sont notamment :
- La polyvalence d'application en ce qui concerne l'engin qui peut être aussi bien un véhicule terrestre - comme une automobile, un train, un tracteur, un engin de manutention -, marin - comme un bateau -, aérien - comme un aéronef-, ou spatial, ou encore, par exemple une grue.
- La polyvalence d'application en ce qui concerne la personne équipée, celle-ci pouvant être aussi bien le pilote de l'engin qu'un passager, selon les définitions données de ces deux termes.
- La flexibilité en ce qui concerne l'engin qui peut n'être pas dédié à la personne équipée, laquelle peut utiliser des engins différents, pour des périodes plus ou moins espacées, ou bien ne pas avoir d'engin dédié.
- La polyvalence d'application en ce que la sécurité envisagée peut concerner une ou plusieurs personnes, une personne pouvant être équipée d'un ou de plusieurs dispositifs médicaux.
- La polyvalence concernant les dispositifs médicaux qui peuvent être implantés ou situés en externe, être à visée thérapeutique et/ou diagnostique.
- La polyvalence en ce qui concerne les dangers ou menaces qui concernent tout à la fois la personne équipée du dispositif médical, le dispositif médical en soi, l'engin ou encore des personnes ou des entités matérielles pouvant être impactées par l'engin.
- La flexibilité en ce sens qu'il peut être envisagé, selon les cas, une alerte unique ou bien une pluralité d'alertes correspondant à des dangers ou menaces plus ou moins élevés.
- La complétude, en ce sens que le problème source de danger ou de menace peut être soit avéré et effectif soit n'être que seulement probable et à venir.
- La prévention du piratage de données sensibles ou physiologiques.
- La prévention d'un transfert massif de données et de leur stockage.

Ci-après, un exposé de l'invention.

Selon un premier aspect, l'invention a pour objet un procédé de sécurité concernant un engin comportant des moyens d'entraînement et au moins un poste pour une personne, dont au moins un poste de service pour une personne servant l'engin - notamment le commandant -, et au moins une personne équipée d'au moins un dispositif médical, l'engin étant conçu pour qu'au moins une personne équipée lui soit associé en se trouvant à un poste de l'engin - notamment au poste de service en vue de servir l'engin -, dans lequel :
- on dispose d'un dispositif médical conçu pour fonctionner en réponse à des données physiologiques de la personne équipée et pour générer, lorsque l'occurrence se présente, un signal d'alerte de dysfonctionnement du dispositif médical *per se* et un signal d'alerte de problème effectif ou déductif concernant l'état médical de la personne équipée,
- on dispose d'un module de sécurité conçu pour être associé structurellement à l'engin, pour recevoir des données générées par le dispositif médical alors que la personne équipée est associée à l'engin, et pour générer des informations en correspondance avec les données qu'il reçoit,
- le dispositif médical et le module de sécurité sont tels que le module de sécurité fonctionne sans recevoir ou stocker les données physiologiques de la personne équipée du dispositif médical rendant ceci non obligatoire,
- alors que la personne équipée est associée à l'engin et qu'à l'engin est associé le module de sécurité, une communication cryptée est établie entre le dispositif médical et le module de sécurité, de sorte que le dispositif médical génère - lorsque justifié - un signal d'alerte de dysfonctionnement et/ou un signal d'alerte de problème médical effectif ou déductif et que le module de sécurité génère au moins une information d'alerte en correspondance avec un signal d'alerte,
- on transmet là au moins une information d'alerte à un opérateur.

Selon une réalisation, on dispose d'un dispositif médical conçu pour générer, en fonction de l'occurrence, le - parmi plusieurs - signal d'alerte de dysfonctionnement du dispositif médical et/ou le - parmi plusieurs - signal d'alerte de problème effectif ou déductif concernant l'état médical de la personne équipée, et dans lequel, à un signal d'alerte parmi plusieurs, est associé un indice du niveau d'alerte.

Selon une réalisation, on dispose d'un dispositif médical qui soit est implanté soit est externe à la personne équipée, à visée thérapeutique et/ou diagnostique.

Selon une réalisation, soit la personne équipée occupe un poste de service de l'engin soit on dispose d'un engin comportant au moins un poste autre que de service, et dans lequel la personne équipée occupe un poste autre que de service.

Selon une réalisation, on dispose d'un dispositif médical et d'un module de sécurité tels que la transmission entre le dispositif médical et le module de sécurité s'établit automatiquement, du seul fait de la présence de la personne équipée à un poste de l'engin et, le cas échéant, de l'activation du module de sécurité.

Selon une réalisation, on dispose d'un dispositif médical et d'un module de sécurité tels que la transmission entre le dispositif médical et le module de sécurité s'établit automatiquement, le cas échéant sans la nécessité obligatoire et systématique d'une opération d'appariement ou le transfert d'un identificateur unique, le cas échéant moyennant un contrôle de la cohérence (en pouvant utiliser le cas échéant un processus d'intelligence artificielle) entre le dispositif médical de la personne équipée et le dispositif médical tel que perçu par le module de sécurité.

Selon une réalisation, on dispose d'un module de sécurité conçu sans mémoire ou sans mémoire durable pour, d'une part, les données physiologiques de la personne équipée, ou pour un identificateur unique destiné à une opération d'appariement, d'autre part, un quelconque signal d'alerte de dysfonctionnement du dispositif médical *per se* ou un signal d'alerte de problème effectif ou déductif concernant l'état médical de la personne équipée.

Selon une réalisation, on dispose soit d'un module de sécurité portatif, associé structurellement à l'engin de façon amovible, on dissocie structurellement le module de sécurité de l'engin lorsque la personne équipée n'est pas à un poste de l'engin et on associe structurellement le module de sécurité à l'engin temporairement lorsque la personne équipée est à un poste de l'engin soit d'un module de sécurité associé structurellement à l'engin à demeure, de façon permanente.

Selon une réalisation, on transmet là au moins une information d'alerte à un opérateur étant choisi comme étant l'un ou plusieurs de : une unité de commande du fonctionnement des moyens d'entraînement de l'engin, une personne se trouvant à un poste de l'engin, système d'alerte distant de l'engin.

Selon une réalisation, on dispose d'un engin comportant une unité de commande du fonctionnement de ses moyens d'entraînement associée fonctionnellement au module se sécurité, on transmet la au moins une information d'alerte à l'unité de commande, et on programme l'unité de commande de sorte qu'elle commande les moyens d'entraînement en fonction de la survenance d'un signal d'alerte de dysfonctionnement du dispositif médical et/ou d'un signal d'alerte de problème médical effectif ou déductif et l'indice du niveau d'alerte dans le cas d'un signal d'alerte parmi plusieurs.

Selon une réalisation, on dispose d'une unité de commande qui soit est programmable et on programme préalablement l'unité de commande selon un scénario ou des scenarii fonction du signal d'alerte soit est préprogrammée selon un scénario ou des scenarii fonction du signal d'alerte.

Selon une réalisation, on dispose d'un moyen sensoriel d'alerte, associé fonctionnellement au module se sécurité, conçu pour générer un signal d'alerte perceptible par une personne se trouvant à un poste de l'engin, comme un moyen visuel, sonore, vibratile ou analogue.

Selon une réalisation, on dispose d'un système d'alerte distant de l'engin, on transmet à distance au système d'alerte la au moins une information d'alerte, on génère une donnée de géolocalisation de l'engin et on transmet à distance au système d'alerte la donnée de géolocalisation.

Selon une réalisation, on détecte la présence d'une personne à un poste de l'engin.

Selon un deuxième aspect, l'invention a pour objet un ensemble de sécurité concernant un engin comportant des moyens d'entraînement et au moins un poste pour une personne, dont au moins un poste de service pour une personne servant l'engin - notamment le commandant -, et au moins une personne équipée d'au moins un dispositif médical, l'engin étant conçu pour qu'au moins une personne équipée lui soit associé en se trouvant à un poste de l'engin - notamment au poste de service en vue de servir l'engin -, pour la mise en œuvre du procédé qui vient d'être décrit, tel que :
- le dispositif médical est conçu pour fonctionner en réponse à des données physiologiques de la personne équipée et pour générer, lorsque l'occurrence se présente, un signal d'alerte de dysfonctionnement du dispositif médical *per* se et un signal d'alerte de problème effectif ou déductif concernant l'état médical de la personne équipée,
- il comporte un module de sécurité conçu pour être associé structurellement à l'engin, pour recevoir des données générées par le dispositif médical alors que la personne équipée est associée à l'engin, et pour générer des informations en correspondance avec les données qu'il reçoit, de sorte que le dispositif médical génère - lorsque justifié - un signal d'alerte de dysfonctionnement et/ou un signal d'alerte de problème médical effectif ou déductif et que le module de sécurité génère au moins une information d'alerte en correspondance avec un signal d'alerte,
- il comporte des moyens de cryptage de la communication entre le dispositif médical et le module de sécurité,
- il comporte des moyens de transmission de là au moins une information d'alerte à un opérateur,
- le dispositif médical et le module de sécurité sont tels que le module de sécurité fonctionne sans recevoir ou stocker les données physiologiques du conducteur équipé du dispositif médical rendant ceci non obligatoire.

Selon une réalisation, le dispositif médical est conçu pour générer, en fonction de l'occurrence, le - parmi plusieurs - signal d'alerte de dysfonctionnement du dispositif médical et/ou le - parmi plusieurs - signal d'alerte de problème effectif ou déductif concernant l'état médical de la personne équipée, et dans lequel, à un signal d'alerte parmi plusieurs, est associé un indice du niveau d'alerte.

Selon une réalisation, le module de sécurité est conçu sans mémoire ou sans mémoire durable pour, d'une part, les données physiologiques de la personne équipée, d'autre part, un quelconque signal d'alerte de dysfonctionnement du dispositif médical *per se* ou un signal d'alerte de problème effectif ou déductif concernant l'état médical de la personne équipée.

Selon une réalisation, le module de sécurité est conçu sans mémoire ou sans mémoire durable pour un identificateur unique et/ou conçu avec un moyen de contrôle de la cohérence entre le dispositif médical de la personne équipée et le dispositif médical tel que perçu par le module de sécurité.

Selon les réalisations, soit le module de sécurité est portatif, associé structurellement à l'engin de façon amovible soit associé structurellement à l'engin à demeure, de façon permanente.

Selon une réalisation, l'ensemble comprend un opérateur choisi comme étant l'un ou plusieurs de : une unité de commande du fonctionnement des moyens d'entraînement de l'engin, une personne se trouvant à un poste de l'engin, système d'alerte distant de l'engin.

Selon une réalisation, l'engin comporte une unité de commande du fonctionnement de ses moyens d'entraînement associée fonctionnellement au module se sécurité, ladite unité de commande étant soit programmable soit préprogrammée.

Selon une réalisation, l'ensemble de sécurité comporte un moyen sensoriel d'alerte, associé fonctionnellement au module se sécurité, conçu pour générer un signal d'alerte perceptible par une personne se trouvant à un poste de l'engin, comme un moyen visuel, sonore, vibratile ou analogue.

Selon une réalisation, l'ensemble de sécurité comporte un système d'alerte distant de l'engin et des moyens de géolocalisationsde l'engin.

Selon une réalisation, les moyens de transmissions entre le dispositif médical, le module de sécurité, les moyens de transmission à un opérateur sont de type sans fil, ou de type filaire.

Selon une réalisation, l'ensemble de sécurité comporte en outre des moyens de détection de la présence d'une personne à un poste de l'engin, associés fonctionnellement au module de sécurité.

Selon les réalisations, l'engin est un véhicule terrestre - comme une automobile, un train, un tracteur, un engin de manutention -, marin - comme un bateau -, aérien - comme un aéronef -, ou spatial.

Selon un troisième aspect, l'invention a pour objet un dispositif médical spécialement destiné à la mise en oeuvre du procédé qui vient d'être décrit, conçu pour fonctionner en réponse à des données physiologiques de la personne équipée et pour générer, lorsque l'occurrence se présente, un signal d'alerte de dysfonctionnement du dispositif médical *per* se et un signal d'alerte de problème effectif ou déductif concernant l'état médical de la personne équipée, le cas échéant parmi plusieurs signaux d'alerte, le dispositif médical étant destiné à être soit implanté soit externe, à visée thérapeutique et/ou diagnostique.

Selon un quatrième aspect, l'invention a pour objet un module de sécurité spécialement destiné à la mise en oeuvre du procédé de sécurité qui vient d'être décrit, conçu pour recevoir des données générées par un dispositif médical et pour générer des informations en correspondance avec les données qu'il reçoit.

L'invention permet un dialogue machine - machine sans requérir une intervention humaine dans son fonctionnement normal et habituel. Si l'invention met en œuvre un dispositif médical et un véhicule, chacun d'eux assure à titre principal sa fonction propre, indépendamment de l'autre, le module de sécurité sert d'intermédiaire entre eux à des fins de sécurité, sans exercer de fonction de diagnostic médical propre et sans exercer de fonction de prise de contrôle du véhicule propre.

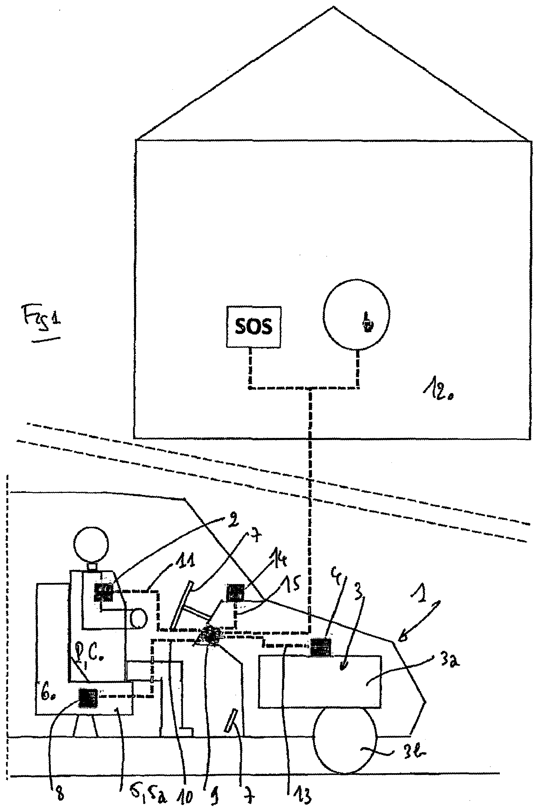

La figure 1 - unique - est une vue purement schématique illustrant l'invention.

On décrit maintenant à titre d'exemple une réalisation particulière possible de l'invention.

Cette réalisation n'est pas exclusive d'autres, l'homme du métier pouvant transposer et adapter les enseignements de la description de la réalisation particulière décrite à d'autres réalisations.

L'invention a pour objet un procédé de sécurité, un ensemble de sécurité spécialement destiné à la mise en oeuvre du procédé, un dispositif médical et un module de sécurité spécialement destinés, l'un comme l'autre, à la mise en œuvre du procédé et, ce faisant, à faire partie de l'ensemble de sécurité. Par suite, la description en ce qu'elle porte sur le procédé de sécurité vaut pour l'ensemble de sécurité ainsi que le dispositif médical et le module de sécurité, et inversement, la description en ce qu'elle porte sur l'ensemble de sécurité, le dispositif médical et le module de sécurité vaut pour le procédé de sécurité.

Comme indiqué, par « sécurité » il faut comprendre une situation aussi objective que possible, reposant sur des conditions d'ordre médical, qui entraîne l'absence ou la minimisation de dangers ou de menaces concernant tout à la fois la personne équipée du dispositif médical, le dispositif médical en soi, l'engin ou encore des personnes ou des entités matérielles pouvant être impactées par l'engin.

Il est entendu que l'invention porte sur tout moyen mentionné, en tant qu'étape opératoire ou d'entité matérielle, ainsi que tout moyen fonctionnellement équivalent.

Il est entendu qu'il n'est pas nécessaire de décrire dans le détail tel ou tel moyen dont il est fait état, dans la mesure où l'indication de son utilité ou de sa fonction ou de l'effet qu'il procure ou de sa mise en œuvre est suffisante pour que l'homme du métier puisse le réaliser.

De façon générale, l'invention vise la sécurité concernant un engin comportant des moyens d'entraînement et au moins un poste pour une personne, dont au moins un poste de service pour une personne servant l'engin - notamment le commandant -, et au moins une personne équipée d'au moins un dispositif médical, l'engin étant conçu pour qu'au moins une personne équipée lui soit associé en se trouvant à un poste de l'engin - notamment au poste de service en vue de servir l'engin -.

Un tel engin peut être aussi bien un véhicule terrestre - comme une automobile, un train, un tracteur, un engin de manutention -, marin - comme un bateau -, aérien - comme un aéronef -, ou spatial, ou encore, par exemple une grue.

La personne équipée peut être aussi bien le pilote de l'engin qu'un passager, selon les définitions données de ces deux termes.

L'engin peut n'être pas dédié à la personne équipée, laquelle peut utiliser des engins différents, pour des périodes plus ou moins espacées, ou bien ne pas avoir d'engin dédié.

La sécurité envisagée peut concerner une ou plusieurs personnes, une personne pouvant être équipée d'un ou de plusieurs dispositifs médicaux.

Les dispositifs médicaux peuvent être implantés ou situés en externe, être à visée thérapeutique et/ou diagnostique.

Les dangers ou menaces visés par la sécurité objet de l'invention concernent tout à la fois la personne équipée du dispositif médical, le dispositif médical en soi, l'engin ou encore des personnes ou des entités matérielles pouvant être impactées par l'engin.

Le problème source de danger ou de menace peut être soit avéré et effectif soit n'être que seulement probable et à venir.

La réalisation particulière décrite vise le cas spécifique d'un véhicule terrestre comme une automobile 1, le conducteur C équipé d'un dispositif médical 2.

L'automobile 1 comporte des moyens d'entraînement 3 comme un moteur 3a auquel sont associés les moyens usuels tels que des roues 3b et des organes de transmission. L'automobile 1 comporte également une unité 4 de commande du fonctionnement de ses moyens d'entraînement 3.

L'automobile 1 comporte au moins un poste 5 pour une personne P, dont au moins un poste de service 5a, à savoir en l'espèce un poste de conduite 5a, pour la personne servant l'automobile, à savoir le conducteur C.

Un poste 5 comprend typiquement un siège 6 et les moyens usuels associés. Ainsi, le poste de conduite 5a comprend un tel siège 6 et des organes de pilotage 7 comme une télécommande, un volant, des manettes, des boutions, des pédales, etc.

Pour une période de déplacement de l'automobile 1, le conducteur C est associé à l'automobile en ce sens qu'il s'installe puis reste assis sur le siège 6 du poste de conduite 5a, de sorte à pouvoir manœuvrer les organes de pilotages 7. Entre les périodes de déplacement, lorsque l'automobile 1 est à l'arrêt et n'est pas utilisée, le conducteur C quitte l'automobile 1 pour d'autres occupations. Il cesse alors d'être associé à l'automobile 1, conformément au sens donné en l'espèce au terme « associé ». Eventuellement, le conducteur C reste associable à l'automobile 1 plus tard.

Le dispositif médical 2 est conçu pour fonctionner en réponse à des données physiologiques du conducteur C et pour générer, lorsque l'occurrence se présente, un signal d'alerte de dysfonctionnement du dispositif médical 2 *per se* et un signal d'alerte de problème effectif ou déductif concernant l'état médical du conducteur C.

Un tel dispositif médical 2 soit est implanté soit est externe au conducteur C. Il est à visée thérapeutique et/ou diagnostique. Un tel dispositif médical 2 peut être par exemple un stimulateur cardiaque, une prothèse, une pompe, un cœur artificiel, un exosquelette, cette liste n'étant pas limitative.

Selon une réalisation, le dispositif médical 2 est conçu pour générer un unique signal d'alerte de dysfonctionnement du dispositif médical et/ou un unique signal d'alerte de problème concernant l'état médical du conducteur C.

Selon une autre réalisation, le dispositif médical 2 est conçu pour générer, en fonction de l'occurrence, un - parmi plusieurs - signal d'alerte de dysfonctionnement du dispositif médical et/ou un - parmi plusieurs - signal d'alerte de problème concernant l'état médical du conducteur C. Dans une telle réalisation, à un signal d'alerte parmi plusieurs, est associé un indice du niveau d'alerte.

Selon une réalisation, le signal d'alerte de problème concernant l'état médical du conducteur C correspond à un problème avéré et effectif. Selon une autre réalisation, il peut n'être que seulement probable et à venir, étant alors le plus souvent précédé de signes annonciateurs.

Dans une réalisation, il est prévu des moyens 8 de détection de la présence du conducteur C au poste de conduite 4a. Par exemple, de tels moyens 8 de détection de présence sont associés au siège du conducteur C.

Ce faisant, le procédé de sécurité comporte une détection de la présence du conducteur C au poste de conduite 5a.

Le procédé met en œuvre et le système de sécurité comprend également un module de sécurité 9.

Le module de sécurité 9 est conçu pour être associé structurellement à l'automobile 1.

Dans le cas où il prévu des moyens 8 de détection de la présence du conducteur C au poste de conduite 5a, il est prévu des moyens de transmission 10 entre les moyens 8 de détection et le module de sécurité 9. Il est ainsi possible d'activer automatiquement le module de sécurité 9, outre que par l'activation par le dispositif médical 2.

Selon une réalisation, le module de sécurité 9 est portatif et associé structurellement à l'automobile 1 de façon amovible. On dissocie alors structurellement le module de sécurité 9 de l'automobile 1 lorsque le conducteur C n'est pas au poste de conduite 5a. Inversement, on associe structurellement le module de sécurité 9 à l'automobile 1 temporairement lorsque le conducteur C est au poste de conduite 5a.

Selon une autre réalisation, le module de sécurité 9 est associé structurellement à l'automobile 1 à demeure, de façon permanente.

Le module de sécurité 9 est conçu pour, d'une part, recevoir des données générées par le dispositif médical 2, alors que le conducteur C est au poste de conduite 5a.

Le module de sécurité 9 est conçu pour, d'autre part, générer des informations en correspondance avec les données qu'il reçoit du dispositif médical 2.

Il est également prévu des moyens de transmission 11 entre le dispositif médical 2 et le module de sécurité 9 et donc le procédé de sécurité comporte une transmission entre le dispositif médical 2 et le module de sécurité 9.

L'ensemble de sécurité comporte également des moyens de cryptage de la communication entre le dispositif médical 2 et le module de sécurité 9. Cette disposition constructive permet d'éviter le piratage des données transmises et assure leur confidentialité.

Avec les moyens précédemment décrits, le dispositif médical 2 génère - lorsque justifié - un signal d'alerte de dysfonctionnement et/ou un signal d'alerte de problème. Et le module de sécurité 9 associé fonctionnellement au dispositif médical 2, comme exposé, génère à son tour au moins une information d'alerte en correspondance avec un signal d'alerte.

Le procédé de sécurité est tel que l'on transmet alors là au moins une information d'alerte à un opérateur, l'ensemble de sécurité comportant des moyens de transmission de ladite information d'alerte à l'opérateur.

L'opérateur dont il est question est choisi comme étant l'un ou plusieurs de: l'unité de commande 4 du fonctionnement des moyens d'entraînement 3 de l'automobile 1, le conducteur C se trouvant au poste de conduite 5a ou un passager de l'automobile 1, ou un système d'alerte 12 distant de l'automobile 1. Et l'ensemble de sécurité comprend un ou plusieurs tels opérateurs 4, C et 12.

Dans le cas où l'opérateur est l'unité de commande 4, l'unité de commande 4 est associée fonctionnellement au module se sécurité 9 par des moyens de transmission 13. Par suite, on transmet l'information d'alerte à l'unité de commande 4.

Dans une réalisation, cette unité de commande 4 est programmable et on la programme préalablement selon un scénario ou des scenarii en fonction de la survenance d'un signal d'alerte de dysfonctionnement du dispositif médical 2 et/ou d'un signal d'alerte de problème médical du conducteur C, le cas échéant en tenant compte de l'indice du niveau d'alerte dans le cas d'un signal d'alerte parmi plusieurs signaux d'alerte.

Dans une autre réalisation, cette unité de commande 4 est préprogrammée selon un tel scénario ou de tels scenarii.

Dans le cas où l'opérateur est le conducteur C se trouvant au poste de conduite 5a ou un passager de l'automobile 1, l'ensemble comporte un moyen sensoriel d'alerte 14, associé fonctionnellement au module se sécurité 9 par des moyens de transmissions 15. Un tel moyen sensoriel d'alerte 14 est conçu pour générer un signal d'alerte perceptible par une personne se trouvant à un poste 4 de l'automobile 1, notamment le conducteur C. Il peut s'agir par exemple d'un moyen visuel, sonore, vibratile ou plus généralement de tout autre moyen analogue. Le procédé se sécurité est alors tel que, moyennant le moyen sensoriel d'alerte 14, on génère un signal d'alerte perceptible par la personne dont il a été question.

Dans le cas où l'opérateur est le conducteur est un système d'alerte 12 distant de l'automobile, l'ensemble de sécurité comporte un tel système d'alerte 12.

Un tel système d'alerte 12 est associé fonctionnellement au module se sécurité 9 par des moyens de transmissions 16. Un tel système d'alerte 12 est conçu de sorte à ce que lui soit transmis à distance l'information d'alerte. De plus, le module de sécurité est agencé de sorte à pouvoir générer directement une donnée de géolocalisation de l'automobile 1 et à la transmettre à distance au système d'alerte 12.

Un tel système d'alerte 12 peut être distant de façon plus ou moins grande de l'automobile 1, par exemple de plusieurs kilomètres ou dizaines de kilomètres.

Un tel système d'alerte 12 peut être par exemple un centre de secours comprenant des cartes, des moyens de diagnostic et le cas échéant du personnel de secours. Ainsi, en cas de danger ou menace correspondant à un signal d'alerte, un tel centre de secours peut agir pour retrouver l'automobile et secourir le conducteur C.

Dans une réalisation, le dispositif médical 2 et le module de sécurité 9 sont tels que la transmission entre le dispositif médical 2 et le module de sécurité 9 s'établit automatiquement, du seul fait de la présence du conducteur sur le siège du poste de conduite 6, grâce aux moyens de détection 8.

Le module de sécurité 9 peut aussi être activé à la suite de la réception d'un signal du dispositif médical 2.

Ainsi, en régime courant, le procédé et le système sont tels qu'il n'est pas nécessaire de prévoir une opération d'appariement ou le transfert d'un identificateur unique.

Toutefois, le cas échéant il est procédé à un contrôle de la cohérence entre le dispositif médical 2 du conducteur C et le dispositif médical tel que perçu par le module de sécurité 9, le module de sécurité 9 étant conçu avec un moyen de contrôle de cette cohérence.

Le dispositif médical 2 et le module de sécurité 9 sont tels que le module de sécurité 9 fonctionne sans recevoir ou stocker les données physiologiques du conducteur C équipé du dispositif médical 2, ce qui assure la confidentialité de ces données. Et de même, le procédé pour fonctionner ne nécessite aucunement l'envoi obligatoire de données physiologiques au module de sécurité.

Ainsi, le module de sécurité 9 est conçu sans mémoire ou sans mémoire durable pour, d'une part, les données physiologiques du conducteur C, ou pour un identificateur unique destiné à une opération d'appariement, d'autre part, un quelconque signal d'alerte de dysfonctionnement du dispositif médical *per se* ou un signal d'alerte de problème concernant l'état médical du conducteur C.

Selon les réalisations, les moyens de transmissions10, 11, 13 et 15 sont de type sans fil, ou de type filaire.

Le cas échéant, ces moyens de transmissions sont compatibles avec un protocole ou une norme concernant les transmissions dans le domaine médical.

## Revendications

1. Procédé de sécurité concernant un engin (1) comportant des moyens d'entraînement (3) et au moins un poste (5) pour une personne, dont au moins un poste (5a) de service pour une personne (C) servant l'engin (1) - notamment le commandant -, et au moins une personne équipée d'au moins un dispositif médical (2), l'engin (1) étant conçu pour qu'au moins une personne équipée lui soit associé en se trouvant à un poste (5) de l'engin (1) - notamment au poste (5a) de service en vue de servir l'engin (1) -, dans lequel :
- on dispose d'un dispositif médical (2) conçu pour fonctionner en réponse à des données physiologiques de la personne équipée et pour générer, lorsque l'occurrence se présente, un signal d'alerte de dysfonctionnement du dispositif médical (2) *per se* et un signal d'alerte de problème effectif ou déductif concernant l'état médical de la personne équipée,
- on dispose d'un module de sécurité (9) conçu pour être associé structurellement à l'engin (1), pour recevoir des données générées par le dispositif médical (2) alors que la personne équipée est associée à l'engin (1), et pour générer des informations en correspondance avec les données qu'il reçoit,
- le dispositif médical (2) et le module de sécurité (9) sont tels que le module de sécurité (9) fonctionne sans recevoir ou stocker les données physiologiques de la personne équipée du dispositif médical (2) rendant ceci non obligatoire,
- alors que la personne équipée est associée à l'engin (1) et qu'à l'engin (1) est associé le module de sécurité (9), une communication cryptée est établie entre le dispositif médical (2) et le module de sécurité (9), de sorte que le dispositif médical (2) génère - lorsque justifié - un signal d'alerte de dysfonctionnement et/ou un signal d'alerte de problème médical effectif ou déductif et que le module de sécurité (9) génère au moins une information d'alerte en correspondance avec un signal d'alerte,
- on transmet là au moins une information d'alerte à un opérateur (4, C, 12).

2. Procédé de sécurité selon la revendication 1, dans lequel on dispose d'un dispositif médical (2) conçu pour générer, en fonction de l'occurrence, le - parmi plusieurs - signal d'alerte de dysfonctionnement du dispositif médical (2) et/ou le - parmi plusieurs - signal d'alerte de problème effectif ou déductif concernant l'état médical de la personne équipée, et dans lequel, à un signal d'alerte parmi plusieurs, est associé un indice du niveau d'alerte.

3. Procédé de sécurité selon l'une des revendications 1 et 2, dans lequel on dispose d'un dispositif médical (2) qui soit est implanté soit est externe à la personne équipée, à visée thérapeutique et/ou diagnostique.

4. Procédé de sécurité selon l'une des revendications 1 à 3, dans lequel soit la personne équipée occupe un poste (5a) de service de l'engin (1) soit on dispose d'un engin (1) comportant au moins un poste (5) autre que de service, et dans lequel la personne équipée occupe un poste (5) autre que de service.

5. Procédé de sécurité selon l'une des revendications 1 à 4, dans lequel on dispose d'un dispositif médical (2) et d'un module de sécurité (9) tels que la transmission entre le dispositif médical (2) et le module de sécurité (9) s'établit automatiquement, du seul fait de la présence de la personne équipée à un poste (5) de l'engin (1) et, le cas échéant, de l'activation du module de sécurité (9).

6. Procédé de sécurité selon l'une des revendications 1 à 5, dans lequel on dispose d'un dispositif médical (2) et d'un module de sécurité (9) tels que la transmission entre le dispositif médical (2) et le module de sécurité (9) s'établit automatiquement, le cas échéant sans la nécessité obligatoire et systématique d'une opération d'appariement ou le transfert d'un identificateur unique, le cas échéant moyennant un contrôle de la cohérence, en pouvant utiliser le cas échéant un processus d'intelligence artificielle, entre le dispositif médical (2) de la personne équipée et le dispositif médical (2) tel que perçu par le module de sécurité (9).

7. Procédé de sécurité selon l'une des revendications 1 à 6, dans lequel on dispose d'un module de sécurité (9) conçu sans mémoire ou sans mémoire durable pour, d'une part, les données physiologiques de la personne équipée, ou pour un identificateur unique destiné à une opération d'appariement, d'autre part, un quelconque signal d'alerte de dysfonctionnement du dispositif médical (2) *per se* ou un signal d'alerte de problème effectif ou déductif concernant l'état médical de la personne équipée.

8. Procédé de sécurité selon l'une des revendications 1 à 7, dans lequel on dispose soit d'un module de sécurité (9) portatif, associé structurellement à l'engin (1) de façon amovible, on dissocie structurellement le module de sécurité (9) de l'engin (1) lorsque la personne équipée n'est pas à un poste (5) de l'engin (1) et on associe structurellement le module de sécurité (9) à l'engin (1) temporairement lorsque la personne équipée est à un poste (5) de l'engin (1) soit d'un module de sécurité (9) associé structurellement à l'engin (1) à demeure, de façon permanente.

9. Procédé de sécurité selon l'une des revendications 1 à 8, dans lequel on transmet la au moins une information d'alerte à un opérateur (4, C, 12) étant choisi comme étant l'un ou plusieurs de: une unité de commande (4) du fonctionnement des moyens d'entraînement (3) de l'engin (1), une personne se trouvant à un poste (5) de l'engin (1), système d'alerte (12) distant de l'engin (1).

10. Procédé de sécurité selon la revendication 9, dans lequel on dispose d'un engin (1) comportant une unité de commande (4) du fonctionnement de ses moyens d'entraînement (3) associée fonctionnellement au module se sécurité (9), on transmet la au moins une information d'alerte à l'unité de commande (4), et on programme l'unité de commande (4) de sorte qu'elle commande les moyens d'entraînement (3) en fonction de la survenance d'un signal d'alerte de dysfonctionnement du dispositif médical (2) et/ou d'un signal d'alerte de problème médical effectif ou déductif et l'indice du niveau d'alerte dans le cas d'un signal d'alerte parmi plusieurs.

11. Procédé de sécurité selon la revendication 10, dans lequel on dispose d'une unité de commande (4) qui soit est programmable et on programme préalablement l'unité de commande (4) selon un scénario ou des scenarii fonction du signal d'alerte soit est préprogrammée selon un scénario ou des scenarii fonction du signal d'alerte.

12. Procédé de sécurité selon la revendication 10, dans lequel on dispose d'un moyen sensoriel d'alerte (14), associé fonctionnellement au module se sécurité (9), conçu pour générer un signal d'alerte perceptible par une personne se trouvant à un poste (5) de l'engin (1), comme un moyen visuel, sonore, vibratile ou analogue.

13. Procédé de sécurité selon la revendication 10, dans lequel on dispose d'un système d'alerte (12) distant de l'engin (1), on transmet à distance au système d'alerte (12) la au moins une information d'alerte, on génère une donnée de géolocalisation de l'engin (1) et on transmet à distance au système d'alerte (12) la donnée de géolocalisation.

14. Procédé de sécurité selon l'une des revendications 1 à 13, dans lequel on détecte la présence d'une personne à un poste (5) de l'engin (1).

15. Ensemble de sécurité concernant un engin (1) comportant des moyens d'entraînement (3) et au moins un poste (5) pour une personne, dont au moins un poste (5a) de service pour une personne servant l'engin (1) - notamment le commandant -, et au moins une personne équipée d'au moins un dispositif médical (2), l'engin (1) étant conçu pour qu'au moins une personne équipée lui soit associé en se trouvant à un poste (5) de l'engin (1) - notamment au poste (5a) de service en vue de servir l'engin (1) -, pour la mise en œuvre du procédé selon l'une des revendications 1 à 14, tel que :
- le dispositif médical (2) est conçu pour fonctionner en réponse à des données physiologiques de la personne équipée et pour générer, lorsque l'occurrence se présente, un signal d'alerte de dysfonctionnement du dispositif médical (2) *per se* et un signal d'alerte de problème effectif ou déductif concernant l'état médical de la personne équipée,
- il comporte un module de sécurité (9) conçu pour être associé structurellement à l'engin (1), pour recevoir des données générées par le dispositif médical (2) alors que la personne équipée est associée à l'engin (1), et pour générer des informations en correspondance avec les données qu'il reçoit, de sorte que le dispositif médical (2) génère - lorsque justifié - un signal d'alerte de dysfonctionnement et/ou un signal d'alerte de problème médical effectif ou déductif et que le module de sécurité (9) génère au moins une information d'alerte en correspondance avec un signal d'alerte,
- il comporte des moyens de cryptage de la communication entre le dispositif médical (2) et le module de sécurité (9),
- il comporte des moyens de transmission de la au moins une information d'alerte à un opérateur (4, C, 12),
- le dispositif médical (2) et le module de sécurité (9) sont tels que le module de sécurité (9) fonctionne sans recevoir ou stocker les données physiologiques du conducteur (C) équipé du dispositif médical (2) rendant ceci non obligatoire.

16. Ensemble de sécurité selon la revendication 15, dans lequel le dispositif médical (2) est conçu pour générer, en fonction de l'occurrence, le - parmi plusieurs - signal d'alerte de dysfonctionnement du dispositif médical (2) et/ou le - parmi plusieurs - signal d'alerte de problème effectif ou déductif concernant l'état médical de la personne équipée, et dans lequel, à un signal d'alerte parmi plusieurs, est associé un indice du niveau d'alerte.

17. Ensemble de sécurité selon l'une des revendications 15 et 16, dans lequel le module de sécurité (9) est conçu sans mémoire ou sans mémoire durable pour, d'une part, les données physiologiques de la personne équipée, d'autre part, un quelconque signal d'alerte de dysfonctionnement du dispositif médical (2) *per se* ou un signal d'alerte de problème effectif ou déductif concernant l'état médical de la personne équipée.

18. Ensemble de sécurité selon l'une des revendications 15 à 17, dans lequel le module de sécurité (9) est conçu sans mémoire ou sans mémoire durable pour un identificateur unique et/ou conçu avec un moyen de contrôle de la cohérence entre le dispositif médical (2) de la personne équipée et le dispositif médical (2) tel que perçu par le module de sécurité (9).

19. Ensemble de sécurité selon l'une des revendications 15 à 18, dans lequel soit le module de sécurité (9) est portatif, associé structurellement à l'engin (1) de façon amovible soit associé structurellement à l'engin (1) à demeure, de façon permanente.

20. Ensemble selon l'une des revendications 15 à 19, qui comprend un opérateur (4, C, 12) choisi comme étant l'un ou plusieurs de : une unité de commande (4) du fonctionnement des moyens d'entraînement (3) de l'engin (1), une personne (C) se trouvant à un poste (5) de l'engin (1), système d'alerte (12) distant de l'engin (1).

21. Ensemble de sécurité selon la revendication 20, dans lequel l'engin (1) comporte une unité de commande (4) du fonctionnement de ses moyens d'entraînement (3) associée fonctionnellement au module se sécurité (9), ladite unité de commande (4) étant soit programmable soit préprogrammée.

22. Ensemble selon la revendication 20, qui comporte un moyen sensoriel d'alerte (14), associé fonctionnellement au module se sécurité (9), conçu pour générer un signal d'alerte perceptible par une personne se trouvant à un poste (5) de l'engin (1), comme un moyen visuel, sonore, vibratile ou analogue.

23. Ensemble de sécurité selon la revendication 20, qui comporte un système d'alerte (12) distant de l'engin (1) et des moyens de géolocalisations de l'engin (1).

24. Ensemble de sécurité selon l'une des revendications 15 à 23, dans lequel les moyens de transmissions entre le dispositif médical (2), le module de sécurité (9), les moyens de transmission à un opérateur (4, C, 12) sont de type sans fil, ou de type filaire.

25. Ensemble de sécurité selon l'une des revendications 15 à 24, qui comporte en outre des moyens de détection (8) de la présence d'une personne à un poste (5) de l'engin (1), associés fonctionnellement au module de sécurité (9).

26. Ensemble de sécurité selon l'une des revendications 15 à 25, dans lequel l'engin (1) est un véhicule terrestre - comme une automobile, un train, un tracteur, un engin (1) de manutention -, marin - comme un bateau -, aérien - comme un aéronef -, ou spatial.

27. Dispositif médical (2) spécialement destiné à la mise en oeuvre du procédé de sécurité selon l'une des revendications 1 à 14, conçu pour fonctionner en réponse à des données physiologiques de la personne équipée et pour générer, lorsque l'occurrence se présente, un signal d'alerte de dysfonctionnement du dispositif médical (2) *per* se et un signal d'alerte de problème effectif ou déductif concernant l'état médical de la personne équipée, le cas échéant parmi plusieurs signaux d'alerte, le dispositif médical (2) étant destiné à être soit implanté soit externe, à visée thérapeutique et/ou diagnostique.

28. Module de sécurité (9) spécialement destiné à la mise en œuvre du procédé de sécurité selon l'une des revendications 1 à 14, conçu pour recevoir des données générées par un dispositif médical (2) et pour générer des informations en correspondance avec les données qu'il reçoit.

## Patentansprüche

1. Sicherheitsverfahren, eine Maschine betreffend (1), die Antriebsmittel (3) aufweist und mindestens einen Posten (5) für eine Person, hierbei mindestens einen Bedienungsposten (5a) für eine Person (C), die die Maschine bedient (1) - vor allem der Kommandant-, und mindestens eine Person, die mit mindestens einer medizinischen Vorrichtung (2) ausgerüstet ist, die Maschine (1) ist so konzipiert, damit ihr wenigstens eine ausgerüstete Person zugeordnet ist und sich an einem Posten (5) der Maschine (1) befindet - vor allem an dem Bedienungsposten (5a) um die Maschine (1) zu bedienen-, in dem :
- man über eine medizinische Vorrichtung (2) verfügt, die konzipiert wurde um in Antwort auf physiologische Daten der ausgerüsteten Person zu funktionieren und, wenn der Fall auftritt, ein Warnsignal wegen Betriebsstörung der medizinischen Vorrichtung (2) durch sich und ein Warnsignal bei einem wirklichen oder herleitbaren Problem bezüglich des gesundheitlichen Zustands der ausgerüsteten Person zu erzeugen,
- man über ein Sicherheitsmodul (9) verfügt, das konzipiert wurde um strukturell mit der Maschine (1) verbunden zu sein, um durch die medizinische Vorrichtung (2) erzeugte Daten zu erhalten während die ausgerüstete Person mit der Maschine (1) verbunden ist, und um Informationen zu erzeugen in Verbindung mit den von ihr erhaltenen Daten
- die medizinische Vorrichtung (2) und das Sicherheitsmodul (9) sind so konzipiert, dass das Sicherheitsmodul (9) funktioniert, ohne die physiologischen Daten der mit der medizinischen Vorrichtung (2) ausgerüsteten Person zu erhalten oder zu speichern, wodurch dieses nicht obligatorisch wird,
- während die ausgerüstete Person mit der Maschine (1) verbunden ist und die Maschine (1) mit dem Sicherheitsmodul (9) verbunden ist, wird eine verschlüsselte Kommunikation zwischen der medizinischen Vorrichtung (2) und dem Sicherheitsmodul (9) hergestellt, so dass die medizinische Vorrichtung (2) -bei Bedarf- ein Warnsignal wegen Betriebsstörung und/oder ein Warnsignal wegen eines wirklichen oder hergeleiteten Gesundheitsproblems erzeugt und dass das Sicherheitsmodul (9) mindestens eine Warninformation in Verbindung mit einem Warnsignal erzeugt.
- man hier mindestens eine Warninformation an einen Bediener überträgt (4, C, 12).

2. Sicherheitsverfahren gemäß Anspruch 1, in dem man über eine medizinische Vorrichtung (2) verfügt, konzipiert um, je nach Fall das - unter mehreren- Warnsigal der Betriebsstörung der medizinischen Vorrichtung (2) und/oder das - unter mehreren - Warnsignal für ein effektives oder hergeleitetes Problem zu erzeugen bezüglich des gesundheitlichen Zustands der ausgerüsteten Person und in dem einem Warnsignal unter mehreren ein Hinweis der Warnstufe zugeordnet ist.

3. Sicherheitsverfahren gemäß eines der Ansprüche 1 und 2, in dem man über eine medizinische Vorrichtung (2) verfügt, die entweder bei der ausgerüsteten Person eingesetzt ist oder sich außerhalb befindet, mit einem therapeutischen und /oder diagnostischen Ziel.

4. Sicherheitsverfahren gemäß eines der Ansprüche 1 bis 3, in dem entweder die ausgerüstete Person einen Bedienungsposten (5a) der Maschine (1) innehat oder man über eine Maschine (1) verfügt, die mindestens einen anderen Posten (5) als den der Bedienung aufweist, und in der die ausgerüstete Person einen anderen Posten (5) als den Bedienungsposten innehat.

5. Sicherheitsverfahren gemäß eines der Ansprüche 1 bis 4, in dem man über eine medizinische Vorrichtung (2) und ein Sicherheitsmodul (9) verfügt, so dass die Übertragung zwischen der medizinischen Vorrichtung (2) und dem Sicherheitsmodul (9) automatisch hergestellt wird, durch die einzige Tatsache der Anwesenheit einer ausgerüsteten Person an einem Posten (5) der Maschine (1) und, gegebenenfalls, der Aktivierung des Sicherheitsmoduls (9).

6. Sicherheitsverfahren gemäß eines der Ansprüche 1 bis 5, in dem man über eine medizinische Vorrichtung (2) und ein Sicherheitsmodul (9) verfügt, so dass die Übertragung zwischen der medizinischen Vorrichtung (2) und dem Sicherheitsmodul (9) automatisch hergestellt wird, gegebenenfalls ohne die obligatorische und systematische Anpassungsoperation oder die Übertragung eines einzigen Identifizierers, gegebenenfalls mit einer Kohärenzkontrolle, indem gegebenenfalls ein Prozess für künstliche Intelligenz benutzt werden kann, zwischen der medizinischen Vorrichtung (2) der ausgerüsteten Person und der medizinischen Vorrichtung (2), so wie es von dem Sicherheitsmodul (9) wahrgenommen wird.

7. Sicherheitsverfahren gemäß eines der Ansprüche 1 bis 6, in dem man über ein Sicherheitsmodul (9) verfügt, das ohne Speicher oder ohne dauerhaften Speicher konzipiert wurde für, einerseits, die physiologischen Daten der ausgerüsteten Person oder für einen einzigen Identifizierer, der für eine Anpassungsoperation bestimmt ist, und andererseits irgendein Warnsigal wegen Betriebsstörung der medizinischen Vorrichtung (2) durch sich oder ein Warnsignal wegen eines effektiven oder hergeleiteten Problems bezüglich des Gesundheitszustandes der ausgerüsteten Person.

8. Sicherheitsverfahren gemäß eines der Ansprüche 1 bis 7, in dem man entweder über ein tragbares Sicherheitsmodul (9) verfügt, das strukturell auf abnehmbare Weise mit der Maschine (1) verbunden ist, man trennt strukturell das Sicherheitsmodul (9) von der Maschine (1), wenn die ausgerüstete Person nicht an einem Posten (5) der Maschine (1) ist und man verbindet strukturell das Sicherheitsmodul (9) vorübergehend mit der Maschine (1), wenn die ausgerüstete Person an einem Posten (5) der Maschine (1) ist, entweder mit einem Sicherheitsmodul (9), das auf Dauer, permanent, strukturell mit der Maschine (1) verbunden ist.

9. Sicherheitsverfahren gemäß eines der Ansprüche 1 bis 8, in dem man mindestens eine Warninformation an einen Bediener (4, C, 12) überträgt, es können eine oder mehrere gewählt werden aus: einer Steuereinheit (4) für das Funktionieren der Antriebsmittel (3) der Maschine (1), einer Person, die sich an einem Posten (5) der Maschine (1) befindet, einem Warnsystem (12), das von der Maschine (1) entfernt ist.

10. Sicherheitsverfahren gemäß des Anspruchs 9, in dem man über eine Maschine (1) verfügt, die eine Steuereinheit (4) für das Funktionieren ihrer Antriebsmittel (3) aufweist und die funktionell mit dem Sicherheitsmodul (9) verbunden ist, man überträgt dort mindestens eine Warninformation an die Steuereinheit (4), und man programmiert die Steuereinheit (4), so dass diese die Antriebsmittel (3) steuert je nach Auftreten eines Warnsignals für eine Betriebsstörung der medizinischen Vorrichtung (2) und/oder ein Warnsignal wegen eines effektiven oder hergeleiteten medizinischen Problems und den Hinweis der Warnstufe im Falle eines Warnsignals unter mehreren.

11. Sicherheitsverfahren gemäß des Anspruchs 10, in dem man über eine Steuereinheit (4) verfügt, die entweder programmierbar ist und man programmiert vorausgehend die Steuereinheit (4) entsprechend eines Ablaufs oder Abläufen je nach Warnsignal, oder die vorprogrammiert ist entsprechend eines Ablaufs oder Abläufen je nach Warnsignal.

12. Sicherheitsverfahren gemäß des Anspruchs 10, in dem man über ein sensorielles Warnsystem (14) verfügt, das funktionell mit dem Sicherheitsmodul (9) verbunden ist, konzipiert, um ein Warnsignal zu erzeugen, das von einer sich an einem Posten (5) der Maschine (1) befindlichen Person wahrgenommen werden kann, als visuelles, akkustisches, vibrierendes oder analoges Mittel.

13. Sicherheitsverfahren gemäß des Anspruchs 10, in dem man über ein Warnsystem (12) verfügt, das von der der Maschine (1) entfernt ist, man überträgt dem Warnsystem (12) aus der Entfernung mindestens eine Warninformation, man erzeugt eine Lokalisierungsgröße der Maschine (1) und man überträgt aus der Entfernung dem Warnsystem (12) die Lokalisierungsgröße.

14. Sicherheitsverfahren gemäß eines der Ansprüche 1 bis 13, in dem man die Anwesenheit einer Person an einem Posten (5) der Maschine (1) detektiert.

15. Sicherheitsganzes, das eine Maschine betrifft (1), die Antriebsmittel (3) aufweist und mindestens einen Posten (5) für eine Person, hierbei mindestens einen Bedienungsposten (5a) für eine Person, die die Maschine bedient (1) - vor allem der Kommandant-, und mindestens eine Person, die mit mindestens einer medizinischen Vorrichtung (2) ausgerüstet ist, die Maschine (1) ist so konzipiert, damit ihr wenigstens eine ausgerüstete Person zugeordnet ist und sich an einem Posten (5) der Maschine (1) befindet - vor allem an dem Bedienungsposten (5a) um die Maschine (1) zu bedienen-, für die Umsetzung des Verfahrens gemäß eines der Ansprüche 1 bis 14, wie :
- die medizinische Vorrichtung (2) ist konzipiert um in Antwort auf physiologische Daten der ausgerüsteten Person zu funktionieren und, wenn der Fall auftritt, ein Warnsignal wegen Betriebsstörung der medizinischen Vorrichtung (2) durch sich und ein Warnsignal bei einem wirklichen oder herleitbaren Problem bezüglich des gesundheitlichen Zustands der ausgerüsteten Person zu erzeugen,
- es weist ein Sicherheitsmodul (9) auf, das konzipiert wurde um strukturell mit der Maschine (1) verbunden zu sein, um durch die medizinische Vorrichtung (2) erzeugte Daten zu erhalten während die ausgerüstete Person mit der Maschine (1) verbunden ist, und um Informationen zu erzeugen in Verbindung mit den von ihr erhaltenen Daten, so dass die medizinische Vorrichtung (2) - wenn dies gerechtfertigt ist - ein Warnsignal wegen Betriebsstörung und/oder ein Warnsignal wegen eines wirklichen oder hergeleiteten Gesundheitsproblems erzeugt und dass das Sicherheitsmodul (9) mindestens eine Warninformation in Verbindung mit einem Warnsignal erzeugt.
- es weist Verschlüsselungsmittel auf für die Kommunikation zwischen der medizinischen Vorrichtung (2) und dem Sicherheitsmodul (9).
- es weist Übertragungsmittel auf von mindestens einer Warninformation an einen Bediener (4, C, 12).
- die medizinische Vorrichtung (2) und das Sicherheitsmodul (9) sind so konzipiert, dass das Sicherheitsmodul (9) funktioniert, ohne die physiologischen Daten der mit der medizinischen Vorrichtung (2) ausgerüsteten Bedienungsperson (C) zu erhalten oder zu speichern, wodurch dieses nicht obligatorisch wird,

16. Sicherheitsganzes gemäß Anspruch 15, in dem die medizinische Vorrichtung (2) konzipiert ist, um, je nach Fall das - unter mehreren- Warnsigal der Betriebsstörung der medizinischen Vorrichtung (2) und/oder das - unter mehreren - Warnsignal für ein effektives oder hergeleitetes Problem zu erzeugen bezüglich des gesundheitlichen Zustands der ausgerüsteten Person und in dem einem Warnsignal unter mehreren ein Hinweis der Warnstufe zugeordnet ist.

17. Sicherheitsganzes gemäß eines der Ansprüche 15 und 16, in dem das Sicherheitsmodul (9) ohne Speicher oder ohne dauerhaften Speicher konzipiert wurde für, einerseits, die physiologischen Daten der ausgerüsteten Person , andererseits irgendein Warnsignal wegen Betriebsstörung der medizinischen Vorrichtung (2) durch sich oder ein Warnsignal wegen eines effektiven oder hergeleiteten Problems bezüglich des Gesundheitszustandes der ausgerüsteten Person.

18. Sicherheitsganzes gemäß eines der Ansprüche 15 bis 17, in dem das Sicherheitsmodul (9) ohne Speicher oder ohne dauerhaften Speicher für einen einzigen Identifizierer konzipiert ist und/oder mit einem Kontrollmittel konzipiert ist für die Kohärenz zwischen der medizinischen Vorrichtung (2) der ausgerüsteten Person und der medizinischen Vorrichtung (2), so wie es von dem Sicherheitsmodul (9) wahrgenommen wird.

19. Sicherheitsganzes gemäß eines der Ansprüche 15 bis 18, in dem entweder das Sicherheitsmodul (9) tragbar ist, das strukturell auf abnehmbare Weise mit der Maschine (1) verbunden ist, oder das auf Dauer, permanent, strukturell mit der Maschine (1) verbunden ist.

20. Sicherheitsganzes gemäß eines der Ansprüche 15 bis 19, das einen Bediener (4, C, 12) aufweist, es können ein oder mehrere gewählt werden aus: einer Steuereinheit (4) für das Funktionieren der Antriebsmittel (3) der Maschine (1), einer Person, die sich an einem Posten (5) der Maschine (1) befindet, einem Warnsystem (12), das von der Maschine (1) entfernt ist.

21. Sicherheitsganzes gemäß des Anspruchs 20, in dem die Maschine (1) eine Steuereinheit (4) für das Funktionieren ihrer Antriebsmittel (3) aufweist, die funktionell mit dem Sicherheitsmodul (9) verbunden ist, die besagte Steuereinheit (4) ist entweder programmierbar oder vorprogrammiert.

22. Sicherheitsganzes gemäß des Anspruchs 20, das ein sensorielles Warnsystem (14) aufweist, das funktionell mit dem Sicherheitsmodul (9) verbunden ist, konzipiert, um ein Warnsignal zu erzeugen, das von einer sich an einem Posten (5) der Maschine (1) befindlichen Person wahrgenommen werden kann, als visuelles, akkustisches, vibrierendes oder analoges Mittel.

23. Sicherheitsganzes gemäß des Anspruchs 20, das ein Warnsystem (12) aufweist, das von der Maschine (1) entfernt ist und Lokalisierungsmittel der Maschine (1)

24. Sicherheitsganzes gemäß eines der Ansprüche 15 bis 23, in dem die Übertragungsmittel zwischen der medizinischen Vorrichtung (2), dem Sicherheitsmodul (9), den Übertragungsmitteln an einen Bediener (4, C, 12) kabellos oder per Kabel erfolgen.

25. Sicherheitsganzes gemäß eines der Ansprüche 15 bis 24, dass außerdem Erkennungsmittel (8) der Anwesenheit einer Person an einem Posten (5) der Maschine (1) aufweist, die funktionell mit dem Sicherheitsmodul (9) verbunden sind.

26. Sicherheitsganzes gemäß eines der Ansprüche 15 bis 25, in dem die Maschine (1) ein Erdfahrzeug ist - wie zum Beispiel ein Auto, ein Zug, ein Traktor, eine Maschine (1) für innerbetriebliche Transportvorgänge - ein Wasserfahrzeug - wie zum Beispiel ein Schiff- in der Luft - wie zum Beispiel ein Flugzeug - oder ein Raumfahrzeug.

27. Mediziniche Vorrichtung (2), die speziell zur Umsetzung des Sicherheitsverfahrens bestimmt ist gemäß eines der Ansprüche 1 bis 14, konzipiert um in Antwort auf physiologische Daten der ausgerüsteten Person zu funktionieren und um, wenn der Fall auftritt, ein Warnsignal für die Betriebsstörung der medizinischen Vorrichtung (2) durch sich zu erzeugen und ein Warnsignal bei einem effektiven oder hergeleiteten Problem bezüglich des Gesundheitszustandes der ausgerüsteten Person, gegebenenfalls unter mehreren Warnsignalen, die medizinische Vorrichtung (2) ist dazu bestimmt, entweder eingesetzt zu werden oder sich ausserhalb zu befinden, mit einem therapeutischen und/oder diagnostischen Ziel.

28. Sicherheitsmodul (9), das speziell zur Umsetzung des Sicherheitsverfahrens bestimmt ist, gemäß eines der Ansprüche 1 bis 14, konzipiert um von einer medizinischen Vorrichtung (2) erzeugte Daten zu erhalten und um Informationen zu erzeugen in Verbindung mit den von ihm erhaltenen Daten.

## Claims

1. Safety process relating to a vehicle (1) incorporating driving means (3) and at least one station (5) for a person, including at least one operating station (5a) for a person (C) to serve the vehicle (1) - in particular the driver in command -, and at least one person fitted with at least one medical device (2), the vehicle (1) being designed so that at least one person fitted with a device is associated with it while being at one station (5) of the vehicle (1) - in particular the operating station (5a) to serve the vehicle (1) -, in which:
- there is a medical device (2) designed to operate in response to physiological data of the person fitted with a device and to generate, when the occurrence arises, an alarm signal warning of malfunction of the medical device (2) *per* se and an alarm signal of an actual or inferred problem relating to the medical condition of the person fitted with the device,
- there is a safety module (9) designed to be structurally associated with the vehicle (1), to receive data generated by the medical device (2) while the person fitted with the device is associated with the vehicle (1), and to generate information matching the data it receives,
- the medical device (2) and the safety module (9) are such that the safety module (9) operates without receiving or storing the physiological data of the person fitted with the medical device (2) making this non-compulsory,
- as the person fitted with the device is associated with the vehicle (1) and the vehicle (1) is associated the safety module (9), encrypted communication is established between the medical device (2) and the safety module (9), so that the medical device (2) generates - when justified - a malfunction alarm signal and/or an alarm signal for an actual or inferred medical problem and the safety module (9) generates at least one alarm notification in line with an alarm signal,
- at least one alarm notification is thereby transferred to an operator (4, C, 12).

2. Safety process based on Claim 1, wherein the medical device (2) is designed to generate, based on the occurrence, the alarm - among several ones - signalling any medical device (2) malfunctioning and/or the alarm - among several ones - signalling an actual or inferred problem concerning the medical status of the person fitted with the device, and where an alarm level index is associated to an alarm signal, among several of them.

3. Safety process according to one of claims 1 and 2, in which a medical device (2) is either implanted in or is external to the person fitted with the device, for therapeutic and/or diagnostic purposes.

4. Safety process according to one of claims 1 to 3, in which either the person fitted with the device occupies a station (5a) operating the vehicle (1) or there is a vehicle (1) comprising at least one station (5) other than the operating station, and in which the person fitted with the device occupies a station (5) other than the operating station.

5. Safety process according to one of claims 1 to 4, wherein the medical device (2) and safety module (9) are configured such that the transfer between the medical device (2) and safety module (9) is automatically established merely due to the presence of the person fitted with the device at a station (5) in the vehicle (1) and activation of the safety module (9) as the case may be.

6. Safety process according to one of claims 1 to 5, wherein the medical device (2) and safety module (9) are configured such that the transfer between the medical device (2) and safety module (9) is automatically established, without the compulsory and systematic necessity of a matching operation or the transfer of a unique identifier as the case may be, by means of a consistency check, using an artificial intelligence process if necessary, between the medical device (2) of the person fitted with the device and the medical device (2) as perceived by the safety module (9).

7. Safety process according to one of claims 1 to 6, in which the safety module (9) is designed with no memory or with no long-term memory for the physiological data of the person with the device, on the one hand, or for a unique identifier intended for a matching operation on the other, any alarm signal reporting any malfunctioning of the medical device (2) *per* se or an alarm signal for an actual or inferred problem concerning the medical condition of the person fitted with a device.

8. Safety process according to one of claims 1 to 7, in which either a portable safety module (9), structurally associated to the vehicle (1) in a removable fashion, the safety module (9) being structurally dislocated from the vehicle (1) when the person fitted with the device is not at a station (5) in the vehicle (1) and the safety module (9) is structurally associated to the vehicle (1) on a temporary basis when the person fitted with the device is at a station (5) in the vehicle (1) or a safety module (9) is structurally associated to the vehicle (1) in a fixed, permanent way.

9. Safety process according to one of claims 1 to 6, wherein at least one alarm notification is transferred to an operator (4, C, 12) selected as one or more from the following list: a control unit (4) for the operation of the vehicle (1) driving means(3), a person at a station (5) in the station of the vehicle (1), a remote alarm system (12) for the vehicle (1).

10. Safety process according to claim 9, in which a vehicle (1) comprising a control unit (4) for the operation of the driving means (3) functionally associated to the safety module (9), at least one alarm notification is transferred to the control unit (4), and the control unit (4) is programmed so that it controls the driving means (3) based on the occurrence of an alarm signal for any malfunctioning of the medical device (2) and/or alarm signal for an actual or inferred medical problem and the alarm level index in the case of an alarm signal among several ones.

11. Safety process according to claim 10, in which the control unit (4) is either programmable - and the control unit (4) is previously programmed based on one or more scenario(s) according to the alarm signal -, or is pre-programmed according to one or more scenario(s) according to the alarm signal.

12. Safety process according to claim 10, in which a sensory alarm system (14), functionally associated to the safety module (9), is designed to generate an alarm signal which can be perceived by a person at a station (5) in the vehicle (1), such as a visual, sound, vibration or comparable system.

13. Safety process according to claim 10, further comprising a remote alarm system (12) in the vehicle (1); at least one alarm notification being remotely transferred to the alarm system (12), geo-location data being generated for the vehicle (1) and the geo-location data are remotely transferred to the alarm system (12).

14. Safety process according to one of claims 1 to 13, in which the presence of a person is detected at a station (5) of the vehicle (1).

15. Safety unit concerning a vehicle (1) incorporating driving means (3) and at least one station (5) for one person, including at least one operating station (5a) for a person serving the vehicle - in particular the driver in command -, and at least one person fitted with at least one medical device (2), the vehicle (1) being designed so that at least one person with a device may be associated to it at a station (5) in the vehicle (1) - in particular at the operating station (5a) to serve the vehicle (1) -, for the implementation of the process according to one of claims 1 to 14, so that:
the medical device (2) is designed to work in response to physiological data relating to the person with a device and to generate, when the occurrence arises, an alarm signal warning of any malfunctioning of the medical device (2) *per* se and an alarm signal warning of any actual or inferred problem with respect to the medical status of the person with a device,
- it includes a safety module (9) designed to be structurally associated to the vehicle (1), to receive data generated by the medical device (2) while the person with a device is associated to the vehicle (1), and to generate information in line with data received so that the medical device (2) may generate - where this is justified - an alarm signal in case of malfunctioning and/or an alarm signal for any actual or inferred medical problem and so that the safety module (9) generates at least one alarm notification matching an alarm signal,
- it comprises means to encrypt communications between the medical device (2) and the safety module (9),
- it comprises means to transfer from there at least one alarm notification to an operator (4, C, 12).
- the medical device (2) and safety module (9) are such that the safety module (9) works without receiving or storing the physiological data of the driver (C) fitted with the medical device (2), which makes this non-compulsory.

16. Safety unit according to claim 15, in which the medical device (2) is designed to generate, depending on the occurrence, the alarm signal - among several of them - warning of any malfunctioning of the medical device (2) and/or the alarm signal - among several of them - warning of an actual or inferred problem concerning the medical status of the person fitted with a device, in which an alarm level index is associated to an alarm signal among several of them.

17. Safety unit according to one of claims 15 and 16, in which the safety module (9) is designed with no memory or no long-term memory for, on the one hand, the physiological data of the person fitted with a device, on the other hand any alarm signal in case of malfunctioning of the medical device (2) *per* se or an alarm signal for any actual or inferred problem regarding the medical condition of the person with a device.

18. Safety unit according to one of claims 15 to 17, in which the safety module (9) is designed with no memory or with no long-term memory for a unique identifier and/or designed with means to ensure that the medical device (2) of the person fitted with a device is consistent with the medical device (2) as perceived by the safety module (9).

19. Safety unit according to one of claims 15 to 18, in which the safety module (9) is either portable, structurally associated to the vehicle (1) in a removable fashion, or structurally associated to the vehicle (1) in a fixed permanent way.

20. Unit according to one of claims 15 to 19, comprising an operator (4, C, 12) selected as being one or several from the following list: a control unit (4) for the operation of the driving means (3) of the vehicle (1), a person (C) at a station (5) in the vehicle (1), a remote alarm system (12) for the vehicle (1).

21. Safety unit according to claim 20, in which the vehicle (1) incorporates a control unit (4) for the operation of its driving means (3) functionally associated to the safety module (9), and this control unit (4) is either programmable or pre-programmed.

22. Unit according to claim 20, comprising a sensory alarm system (14), functionally associated to the safety module (9), designed to generate an alarm signal perceptible by a person at a station (5) in the vehicle (1), such as a visual, sound, vibration or comparable system.

23. Safety unit according to claim 20, comprising a remote alarm system (12) for the vehicle (1) and a vehicle (1) geo-location system.

24. Safety unit according to one of claims 15 to 23, in which communication means between the medical device (2), safety module (9), transfer means to an operator (4, C, 12) are wireless or wired systems.

25. Safety unit according to one of claims 15 to 24, further including means (8) for detecting the presence of a person at a station (5) in the vehicle (1), functionally associated to the safety module (9).

26. Safety assembly according to one of claims 15 to 25, in which the vehicle (1) is a land vehicle - such as a motor car, a train, tractor, handling vehicle (1) -, sea vehicle - such as a boat -, air vehicle - such as an aircraft -, or space craft.

27. Medical device (2) specially designed for the implementation of the safety process according to one of claims 1 to 14, designed to work in response to the physiological data of the person fitted with a device and to trigger, when the occurrence arises, an alarm signal for any malfunctioning of the medical device (2) *per* se and an alarm signal for any actual or inferred problem with respect to the medical condition of the person with a device, as the case may be, among several alarm signals, and the medical device (2) is intended to be either implanted or to be external, and be used for therapeutic and/or diagnostic purposes.

28. Safety module (9) specially designed for the implementation of the safety process based on one of claims 1 to 14, designed to receive data generated by a medical device (2) and to generate information in line with the data received.
